Europäisches Patentamt

European Patent Office

Office européen des brevets

(19)

(11) Numéro de publication : **0 188 146**
**B1**

(12) **FASCICULE DE BREVET EUROPÉEN**

(45) Date de publication du fascicule du brevet :
28.03.90

(51) Int. Cl.⁵ : **A 61 L 15/00, C 08 F 251/02**

(21) Numéro de dépôt : 85402382.7

(22) Date de dépôt : 03.12.85

(54) Procédé de préparation de cellulose modifiée lui conferant des propriétés absorbantes améliorées.

(30) Priorité : 14.12.84 FR 8419123

(43) Date de publication de la demande :
23.07.86 Bulletin 86/30

(45) Mention de la délivrance du brevet :
28.03.90 Bulletin 90/13

(84) Etats contractants désignés :
AT BE CH DE FR GB IT LI NL SE

(56) Documents cités :
FR—A— 2 155 661
FR—A— 2 276 423
US—A— 3 997 647
US—A— 4 036 588
US—A— 4 151 130
JOURNAL OF APPLIED POLYMER SCIENCE, vol. 19, 1975, pages 1257-1268, John Wiley & Sons, Inc.; P. LEPOUTRE et al.: "Grafting acrylonitrile onto wood pulp: influence of process variables"

(73) Titulaire : **LA CELLULOSE DU PIN**
**353, bd du Président Wilson**
**F-33200 Bordeaux (FR)**

(72) Inventeur : **Armagnacq, Sylviane**
**3 Allée des Orious Gazinet**
**F-33610 Cestas (FR)**
Inventeur : **Bol, Michel**
**11 Rue Guy Celerier**
**F-33148 Taussat (FR)**

(74) Mandataire : **Muller, René et al**
**SAINT-GOBAIN RECHERCHE 39, quai Lucien Lefranc**
**F-93304 Aubervilliers (FR)**

## Description

La présente invention concerne la préparation de produits à base de dérivés de cellulose ayant des caractéristiques absorbantes améliorées aussi bien vis-à-vis de l'eau que vis-à-vis des liquides physiologiques, par exemple l'urine ou le sang.

Le marché des produits absorbants à jeter, comme par exemple les couches ou les pansements chirurgicaux, s'est rapidement développé au cours de ces dernières années. Pour répondre à une demande croissante et toujours plus exigeante, on a alors cherché à mettre au point des produits qui, sous une masse réduite, présentent une capacité d'absorption encore plus élevée.

Jusqu'à présent, les pâtes de bois classiques ont fourni une grande partie des matériaux absorbants, grâce à leur structure fibreuse dont la capacité et la rapidité d'absorption sont connues. Ces matériaux comme par exemple les pâtes fluff qui sont des pâtes cellulosiques, obtenues à partir du bois et blanchies puis conditionnées pour des usages sanitaires, ont une capacité d'absorption de l'ordre de 10 à 15 g d'eau par gramme de pâte.

Mais on cherche à obtenir des produits dont les capacités d'absorption sont encore meilleures.

On a déjà proposé, à cet effet, d'associer à une structure fibreuse cellulosique des polymères hydrophiles.

On sait par exemple préparer des matériaux composites formés de strates alternées de matelas fibreux cellulosiques et de couches de polymères, comme le polyacrylate de sodium, sous forme de poudre. On utilise généralement une couche de poudre entre deux matelas cellulosiques. Une telle structure présente des propriétés d'absorption et de rétention d'eau ou de liquide physiologiques améliorées, mais la présence de poudre peut être considérée comme un inconvénient dans la manipulation du produit, surtout dans le domaine de l'hygiène.

On s'est alors orienté vers la préparation de dérivés de cellulose obtenus par greffage et polymérisation in situ de monomères sur les fibres cellulosiques d'une pâte dérivée du bois ou d'un autre matériau cellulosique, de façon à disposer d'un matériau présentant des capacités d'absorption et de rétention améliorées tout en étant facilement manipulable.

Des matériaux cellulosiques modifiés par des polymères contenant des groupements carboxyliques ou des groupements fonctionnels hydrolysables sont maintenant connus dans la technique. Un mode opératoire de greffage d'un monomère à insaturation oléfinique, contenant des groupements carboxyliques (par exemple un acide ou un sel acrylique) ou des groupements fonctionnels hydrolysables, par exemple l'acrylate d'éthyle, l'acrylate de butyle ou l'acrylonitrile, et de polymérisation in situ, est décrit par exemple dans le brevet US 3 194 724. Par ailleurs, la publication de brevet français FR 2 276 423 décrit un mode opératoire de préparation d'une pâte de bois modifiée par greffage, notamment de polyacrylonitrile et ayant des propriétés d'absorption d'eau. Selon le procédé décrit, le greffage proprement dit est suivi d'une hydrolyse des fibres greffées, d'un lavage ultérieur à l'eau, ce qui donne au produit son état de gonflement maximum, d'une acidification à un pH donnant au produit son état de gonflement minimum, de la transformation du produit dans sa forme sel, par addition de soude en présence d'un mélange eau-méthanol, suivi d'un lavage à l'alcool pur pour éliminer la soude résiduelle, et d'un séchage. Le matériau cellulosique sec est alors utilisé sous forme de feuilles pour former un produit absorbant.

Mais les conditions indiquées dans le document FR 2 276 423 présentent un certain nombre d'inconvénients pour la fabrication industrielle de matériaux présentant des capacités d'absorption et de rétention améliorées vis-à-vis de l'eau ou de liquides physiologiques.

Suivant les applications données à la feuille absorbante de cellulose modifiée obtenue, il convient qu'elle présente ou non des caractéristiques spécifiques.

On peut par exemple envisager d'utiliser la feuille telle quelle, en lui faisant conserver sa structure fibreuse.

On peut encore envisager la préparation d'un produit absorbant composé d'un mélange d'un produit fluff classique et d'une certaine quantité de cellulose absorbante modifiée qui a été défibrée pour mieux se prêter au mélange. Dans cette application, il est nécessaire que la feuille puisse se défibrer facilement, en donnant des fibres cellulosiques de longueur convenable de façon à ne pas abaisser les caractéristiques d'absorption du produit.

Cette notion d'aptitude au défibrage d'un produit fibreux est particulièrement importante pour différencier le comportement de pâtes cellulosiques.

Or on a constaté que les feuilles de cellulose obtenues par la mise en œuvre du procédé décrit dans la publication française de brevet FR 2 276 423, si elles présentent des capacités d'absorption d'eau ou de liquide physiologique convenables, sous forme de feuilles, ont par ailleurs un aspect dur et cassant : cette caractéristique implique une mauvaise aptitude au défibrage qui se traduit par l'obtention de fibres trop courtes après l'étape de défibrage. Du fait de cette courte longueur de fibres, la capacité d'absorption par capillarité est abaissée, ce qui, tout en ne modifiant pas la capacité d'absorption par gonflement, abaisse la capacité d'absorption totale. On est alors contraint de limiter l'utilisation d'un tel produit à une application sous forme de feuille non défibrée.

L'invention vise à fournir un produit fibreux à base de cellulose modifiée, obtenu par greffage sur un matériau cellulosique d'un groupement hydrolysable et qui ne présente pas ces inconvénients.

Plus précisément, elle vise à fournir des produits fibreux cellulosiques secs, ayant une capacité

2

d'absorption, et de rétention d'eau ou de liquide physiologique améliorées par rapport aux matériaux connus, qui conservent leur structure fibreuse tout en étant aptes aux défibrages. Ces produits peuvent être conditionnés sous forme de feuilles continues ou discontinues, ou encore en balles.

L'invention concerne un procédé pour la préparation des produits fibreux cellulosiques, secs, par greffage d'un monomère polymérisable à insaturation oléfinique ayant des groupements fonctionnels hydrolysables, sur un matériau fibreux cellulosiques, notamment une pâte cellulosique, qui constitue un perfectionnement des procédés connus.

Conformément à l'invention, le procédé comprend la succession d'étapes suivantes : on active la cellulose contenue dans la pâte cellulosique, on greffe le monomère polymérisable à insaturation oléfinique ayant des groupements fonctionnels hydrolysables sur la cellulose, on hydrolyse la pâte cellulosique greffée avec un alcali, on lave le produit à l'eau jusqu'à obtention d'un état de gonflement maximum, on acidifie le produit à un pH tel qu'après élimination d'eau, il se trouve dans son état de gonflement minimum, on transforme le produit en sa forme sel, en présence d'un liquide miscible à l'eau, et on le sèche, le procédé se caractérisant en ce que la siccité initiale de la cellulose lors du greffage est suffisante et d'au moins 20 % environ pour obtenir un taux de greffage de l'ordre de 200 %, la transformation du produit en sa forme sel est conduite sous une agitation suffisante pour éviter l'agglomération des fibres entre elles, et on fait en sorte que juste avant l'étape de séchage, la quantité d'eau n'excède pas environ 10 % en volume de la phase liquide.

Les produits fibreux obtenus par le procédé selon l'invention présentent une capacité d'absorption d'eau mesurée sous une pression de 25 mbars, supérieure à environ 35 g par gramme de produit et une capacité d'absorption de liquide physiologique, mesurée sous une pression de 25 mbars, représenté par une solution de 1 % de Nacl, supérieure à environ 15 g par gramme de produit ainsi qu'une vitesse d'absorption, mesurable en même temps que la capacité d'absorption, supérieure à environ 15 g par minute et par gramme de produit pour la solution saline. Ils présentent également une aptitude au défibrage au moins cinq fois, et de préférence dix fois supérieure à celle des produits usuels.

L'invention concerne également des produits d'hygiène absorbants qui comprennent des produits fibreux obtenus selon le procédé décrit ci-dessus.

La réunion de toutes les conditions énoncées ci-dessus fournit un produit présentant une aptitude satisfaisante au défibrage.

Outre le document déjà cité FR 2 276 423 qui ne décrit pas ces opérations essentielles pour l'obtention d'un produit présentant une bonne aptitude au défibrage, d'autres produits et procédés pour l'obtention de produits superabsorbants ont été décrits. Mais aucun des documents cités ci-après ne décrit les opérations essentielles du procédé conduisant à un produit présentant à la fois de bonnes propriétés d'absorption et une bonne aptitude au défibrage. On peut citer par exemple les documents FR-A-2 155 661, US-A-3 997 647, US-A-4 036 588 et US-A-4 151 130.

Aucun de ces documents n'oriente l'homme de métier vers un choix judicieux d'opérations conduisant à un produit ayant une bonne aptitude au défibrage.

Suivant une première variante du procédé selon l'invention, entre l'étape de transformation du produit en sa forme sel et l'étape de séchage, pour que la quantité d'eau exprimée en volume n'excède pas 10 % environ de la phase liquide avant l'étape de séchage, on effectue un échange complet du liquide réactionnel avec un liquide miscible à l'eau.

Suivant une seconde variante particulièrement avantageuse du procédé on modifie le procédé tel que décrit précédemment de la façon suivante : l'addition du liquide miscible à l'eau est remplacée par un déplacement par le liquide miscible à l'eau, de l'eau acidifiée présente du fait de l'acidification suivie d'une dilution du milieu avec ledit liquide miscible à l'eau.

Cette variante présente l'avantage d'exiger une moindre quantité de liquide miscible à l'eau.

Suivant une caractéristique avantageuse de l'invention, la transformation du produit en sa forme sel, en présence d'un liquide miscible à l'eau se fait par addition d'ammoniaque.

L'utilisation d'ammoniaque présente l'avantage, dans la mise en œuvre du procédé selon l'invention, d'éviter une étape supplémentaire de lavage, pour éliminer les quantités de réactif en excès. L'ammoniaque peut en effet être directement éliminé lors du séchage, grâce à sa nature très volatile, ce qui n'est pas le cas de la soude, qui a déjà été proposée pour réaliser cette étape dans le brevet cité ci-dessus.

Avantageusement, l'ammoniaque nécessaire à cette étape provient d'une étape antérieure du procédé, ce qui évite tout apport supplémentaire de réactif. En effet, l'hydrolyse des fibres greffées et éventuellement de l'homopolymère également formé, conduit à l'obtention d'un grand excès d'ammoniaque, et la quantité dégagée mise en solution dans l'alcool est suffisante pour transformer par la suite le produit fibreux en sa forme sel.

Le liquide miscible à l'eau joue un rôle de dispersant pour éviter un regroupement des fibres. Ce peut être par exemple un alcool comme le méthanol, l'éthanol ou l'isopropanol.

Le matériau cellulosique de départ utilisé peut être de la pâte de bois, de préférence de la pâte chimique blanchie, de la pâte de bois hydrolysée ou de la rayonne, ou encore du coton.

Les monomères polymérisables à insaturation oléfinique qui ont des groupements fonctionnels hydrolysables peuvent être tous les monomères connus pour leur aptitude au greffage sur de la cellulose et présentant une meilleure aptitude au greffage sur la cellulose qu'à la formation d'homopolymère dans le milieu considéré. Ils comprennent essentiellement les monomères vinyliques et acryliques et leurs

3

dérivés insolubles ou très peu solubles dans l'eau. On peut citer par exemple l'acrylonitrile, le méthacrylonitrile...

Pour obtenir des taux de greffage du monomère polymérisable de l'ordre de 200 %, désirables pour l'obtention de produits conformes à l'invention, une siccité initiale de la cellulose au début du greffage, avant introduction du monomère polymérisable, de l'ordre de 20 % au moins convient. Avec cette condition réalisée conjointement à une agitation convenable de la suspension de fibres au cours de la transformation des groupements greffés sur les fibres cellulosiques en leur forme sel, et à une teneur en eau qui n'excède pas environ 10 % en volume de la phase liquide avant le séchage, le produit final peut être facilement défibré lorsque cela s'avère nécessaire ou utile.

La réaction de greffage peut être effectuée à une température aux alentours de la température de reflux de l'azéotrope eau-acrylonitrile, c'est-à-dire à une température de l'ordre de 70 °C.

On peut théoriquement utiliser tout procédé de greffage, utilisant des initiateurs chimiques ou à rayonnement.

On a toutefois constaté qu'un initiateur chimique d'un type particulier présentait à la fois des modalités d'utilisation simples et un prix de revient modéré. Selon cet aspect de l'invention, la cellulose est activée à l'aide d'un oxydant décomposable à la chaleur et introduit en une seule étape dans le matériau cellulosique à activer. Un tel initiateur est par exemple le persulfate d'ammonium, de potassium ou de sodium. L'utilisation d'un tel initiateur de polymérisation présente l'avantage supplémentaire de fournir un produit blanc, dont la teinte ne s'altère pas au cours du temps. On a constaté en outre que la blancheur du produit pouvait être améliorée en détruisant le persulfate en excès à la fin du greffage par un réducteur, par exemple le bisulfite de sodium. C'est pourquoi, suivant une caractéristique supplémentaire de l'invention, on introduit un tel réducteur après le greffage et avant l'hydrolyse.

L'hydrolyse qui fait suite au greffage peut être faite avec NaOH, KOH, LiOH, et de façon générale, avec une quelconque base forte. La concentration en base est de l'ordre de 3 % en équivalent NaOH pour éviter une éventuelle dégradation du squelette cellulosique.

Les températures d'hydrolyse peuvent être comprises entre la température ambiante et la température d'ébullition de l'eau, et la durée peut varier de quelques minutes à plusieurs jours. Ainsi à 100 °C, la réaction d'hydrolyse dure environ 1 heure.

Pour sécher le superabsorbant, alors que les groupements greffés se trouvent sous leur forme sel, la quantité d'eau étant limitée à 10 % environ en volume, on peut procéder de deux façons distinctes suivant la forme sous laquelle on souhaite conditionner le produit. En vue d'un conditionnement en bobine, c'est-à-dire sous forme de feuilles continues, on met le produit en feuille dès qu'il a été transformé en sa forme sel. La mise en feuille est suivie d'un séchage sur une machine du type machine à papier, le séchage s'accompagnant d'une évaporation du liquide miscible à l'eau contenu dans le mélange réactionnel et de sa récupération. En vue d'un conditionnement en balles, on essore le produit tout de suite après l'obtention de la forme sel, de préférence jusqu'à atteindre une siccité de l'ordre de 15 % à 25 %, puis on sèche le produit obtenu en utilisant par exemple la technique dite du flash drying.

Dans une forme préférée, les conditions opératoires suivantes sont réunies :

l'activateur de la cellulose est le persulfate d'ammonium,

la siccité de la cellulose au début du greffage est de 20 %,

le monomère greffé est l'acrylonitrile,

le lavage à l'eau se fait jusqu'à obtenir une siccité de 2,5 %,

l'acidification du produit est conduite jusqu'à un pH de 3,

le produit acidifié est essoré jusqu'à obtenir une siccité de l'ordre de 20 à 25 %,

l'eau acidifiée est déplacée à l'aide d'éthanol,

le milieu est dilué à l'éthanol.

D'autres caractéristiques et avantages de l'invention apparaîtront au cours de la description suivante d'un exemple détaillé de mise en œuvre de l'invention.

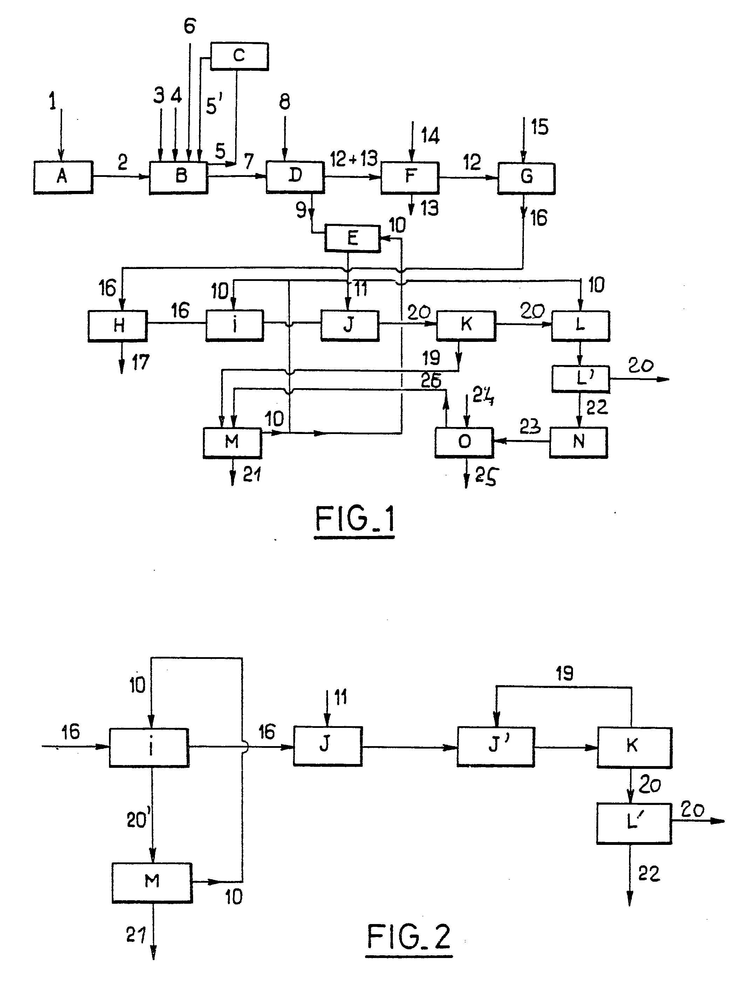

On décrira ci-après deux exemples de réalisation de l'invention dans lequel le monomère greffé est l'acrylonitrile. La description sera faite en relation avec les figures 1 et 2 qui représentent la succession des étapes du procédé.

Les quantités indiquées conduisent à l'obtention d'une tonne de superabsorbant « bone dry », c'est-à-dire sec à 100 %. Les concentrations sont exprimées en siccité, la siccité désignant le pourcentage en poids du produit sec par rapport à un produit brut, lorsque le produit brut contient un composé volatile.

Les différentes étapes du procédé sont matérialisées par des lettres.

On décrira également deux exemples comparatifs qui mettront en évidence les améliorations apportées par l'invention.

Les produits absorbants peuvent être caractérisés par un certain nombre de valeurs qui mettent en évidence leurs propriétés et donc également les améliorations apportées par l'invention : ces caractéristiques sont la valeur de rétention en eau (VRE), la valeur de rétention en sel (VRS), la capacité d'absorption et l'aptitude au défibrage. On indique ci-après les méthodes utilisées pour établir ces caractéristiques :

1. VRE et VRS

Le produit est gonflé dans l'eau ou dans la solution saline, il est ensuite centrifugé pendant 1 minute et demie à 1 250 g. La quantité d'eau ou de solution saline restant dans le produit est déterminée par séchage à 105 °C jusqu'à poids constant.

2. Capacité d'absorption d'eau ou de liquide physiologique

C'est la qualité essentielle d'une pâte pour articles d'hygiène absorbants. Elle se manifeste selon deux critères : la capacité d'absorption et la vitesse d'absorption du matelas fibreux. Une vitesse d'absorption élevée est fortement souhaitable pour des applications à l'hygiène. On obtient la capacité d'absorption de la façon suivante : on place une petite quantité de produit superabsorbant sur une pièce cylindrique dont le fond est recouvert d'une toile mécanique. On pose sur le produit un poids de façon à exercer une pression constante de 25 mbars. La pièce cylindrique est reliée par l'intermédiaire d'un tuyau souple à une burette graduée contenant le liquide, eau ou solution saline à 1 % de Nacl. La toile est en contact permanent avec le liquide. On ouvre la burette jusqu'à ce que le niveau soit stable, ce qui correspond à l'absorption maximale de liquide par le produit.

En mesurant toutes les 15 secondes la quantité de liquide absorbée, on détermine la vitesse d'absorption.

3. Aptitude au défibrage

En l'absence de norme précise sur ce point, la demanderesse a défini une méthode fiable, déterminant le temps nécessaire pour obtenir une individualisation complète des fibres contenues dans un échantillon.

Pour déterminer l'aptitude au défibrage d'une pâte cellulosique, on procède de la façon suivante : de la pâte à tester est climatisée 24 heures à 20 °C et on découpe au massicot un lot de carrés de 2 × 2 cm. L'appareillage utilisé pour la mesure consiste en un bol à fond hémisphérique, du type utilisé dans les moulins à café électriques, équipé d'une hélice robuste en acier inoxydable dont les dimensions et la forme assurent un balayage et un brassage efficaces. L'hélice est vissée sur un axe équipé de roulements à billes, muni d'un cône d'entraînement pouvant se positionner sur une base moteur de 800 W tournant à 20 500 t/mn. Un couvercle translucide évite les projections et permet de suivre les opérations.

Pour la mesure, on pèse 3,0 ± 0,05 g de carrés de pâte que l'on dispose au fond du bol. Le bol fermé, posé sur la base moteur, est actionné par périodes de 10 secondes, alternant un arrêt de 5 secondes pour éviter un échauffement excessif de la pâte, jusqu'à ce que la séparation des fibres soit jugée suffisante. On tire alors une feuille avec la charge défibrée, sur une formette de laboratoire, on presse et on sèche.

L'examen de la feuille permet de déceler le défaut éventuel de défibrage, grâce à la présence de grains, pastilles. On défibre alors une nouvelle charge un peu plus longtemps, jusqu'à obtention d'un épais parfait. L'indice de défibrabilité correspond à la durée totale de défibrage nécessaire pour obtenir ce résultat. Plus la durée est longue, moins bonne est l'aptitude au défibrage de la pâte.

Exemple comparatif 1

On prépare une pâte fluff usuelle pour la fabrication d'articles cellulosiques absorbants.
Les mesures des propriétés du produit absorbant ont donné les résultats suivants :

| | Indice de défibrabilité (secondes) | VRE (g/g de produit) | VRS (g/g de produit) | Capacité d'absorption d'une solution saline (g/g de produit) |
|---|---|---|---|---|
| fluff | 25–50 | 1 | 1 | 10 |

Exemple comparatif 2

On prépare un matériau cellulosique absorbant selon les données de la publication de brevet française 2 276 423.
On observe un très mauvais indice de défibrabilité supérieur à 50 secondes.

Exemple 1

Préparation de feuilles continues de superabsorbant par greffage de polyacrylonitrile.

On amène 0,34 tonne air dry de pâte chimique blanchie 1 de type fluff en A, où la pâte est défibrée. La pâte cellulosique défibrée 2 est amenée en B, dans un réacteur maintenu sous agitation. En même temps que la pâte cellulosique défibrée, on introduit en B 5 kg de persulfate d'ammonium 3, dilué dans 0,9 m³ d'eau et 730 kg de monomère acrylonitrile 4, nécessaires au greffage du polymère acrylonitrile sur les fibres cellulosiques, si bien qu'au début du greffage, après introduction de l'acrylonitrile, la siccité est de l'ordre de 20 %. Le réacteur B est porté en 5 minutes environ à une température correspondant à la

5

température d'ébullition de l'azéotrope eau-acrylonitrile, soit environ 70 °C. L'azéotrope 5 qui se dégage est condensé en C puis réintroduit sous forme liquide 5' en B. Au bout de 45 minutes environ, on arrête l'introduction de l'azéotrope liquide dans le réacteur B pour le stocker. La quantité stockée sera introduite ultérieurement en B pour la fabrication d'une nouvelle quantité de superabsorbant. Après élimination de l'acrylonitrile en excès, on ajoute 8 m³ d'une solution 6 de bisulfite de sodium à 0,38 % de matière sèche. On laisse réagir pendant 10 minutes. La pâte greffée 7 est introduite en case D dans un second réacteur pour l'opération d'hydrolyse. Simultanément, on introduit 15 m³ d'une solution alcaline à 45 g par litre de NaOH 8. La réaction d'hydrolyse s'effectue à 100 °C pendant 30 minutes. Au cours de la réaction, de l'ammoniaque 9 se dégage et est absorbé en case E sur une colonne par de l'éthanol à 95 % 10. La solution eau-alcool-ammoniaque 11 produite est utilisée ultérieurement en case J pour la neutralisation de la forme acide du produit absorbant 16. Après la réaction d'hydrolyse, on effectue en F une séparation et un lavage de la phase fibreuse absorbante produite 12 pour éliminer les jus basiques et toutes les molécules non greffées ou dissoutes 13. Pour ce lavage, on introduit en case F les quantités d'eau nécessaires 14 pour obtenir le produit fibreux dans sa phase de gonflement maximal 12, correspondant à une siccité de l'ordre de 2,5 %, les jus produits 13 étant éliminés. Le produit fibreux 12 est introduit en case G pour une acidification à pH3 conduisant à sa transformation sous forme acide 16 dans son état de gonflement minimal. On introduit donc en case G 8 m³ d'eau acidifiée à 35 g/l de $H_2SO_4$ 15. Le produit obtenu 16 est épaissi à 20 % de siccité environ en case H, avec rejet de l'eau et des résidus tels que $H_2SO_4$ et $Na_2SO_4$ 17. Au produit 16, on ajoute 6 m³ environ d'éthanol à 95 % 10 en case I.

Le superabsorbant sous forme acide 16 dans un milieu alcoolique est neutralisé en J, où il est transformé en sa forme sel, par un apport du mélange éthanol à 95 % et ammoniaque 11 provenant de la case E. La neutralisation se fait sous une agitation suffisante pour éviter l'agglomération des fibres superabsorbantes entre elles.

Le superabsorbant neutralisé 18 est épaissi en case K à une siccité de l'ordre de 20 %. On effectue un échange complet du liquide en L par de l'éthanol à 95 % 10 de façon à ce que la quantité d'eau dans le produit n'excède pas 10 % en volume environ de la phase liquide. Le matériau est séché ensuite en case L' par la technique du flash drying. L'alcool récupéré 19 en case K est recyclé vers la colonne de distillation en case M. Le résidu 21 de distillation qui contient essentiellement de l'eau, des traces d'alcool, du $Na_2SO_4$, du $(NH_4)_2SO_4$ soluble dans l'eau, et des traces de $H_2SO_4$ est rejeté. Les vapeurs 22 dues au séchage en case L' sont condensées en case N. Le liquide produit 23 est neutralisé en O par ajout d'acide sulfurique 24 et conduit à la précipitation de $(NH_4)_2SO_4$ 25, qui est éliminé du circuit. L'alcool et l'eau résiduelle 26 sont envoyés en case M pour l'opération de distillation.

L'alcool 10 récupéré après l'opération de distillation en case M est recyclé en partie vers la colonne d'absorption E. Une autre partie est ajoutée en case I et L comme indiqué précédemment.

On obtient ainsi une tonne de superabsorbant 20 sec sous forme fibreuse qui peut être utilisée pour la fabrication de produits d'hygiène.

## Exemple 2

On procède comme dans l'exemple 1 en modifiant le procédé selon la succession d'étapes indiquées à la figure 2. Les modifications apparaissent au niveau de la case I : au lieu d'ajouter l'éthanol en case I, on déplace l'eau acidifiée par 6 m³ d'éthanol à 95 % 10. Le superabsorbant sous forme acide 16 dans un milieu alcoolique est neutralisé en J où il est transformé en sa forme sel sous agitation. Le résidu 20' du lavage à l'éthanol en I est envoyé vers la colonne de distillation en case M. Le superabsorbant est dilué en case J' dans de l'éthanol à 95 % 19 à 5 à 10 g par litre de produit.

Il est mis en feuille en case K et séché en case L', les deux opérations étant effectuées dans une machine à papier convenable. L'éthanol liquide 19 récupéré en case K est recyclé en case J' pour l'opération de dilution. L'éthanol vapeur 22 dû au séchage en case L est condensé en case N (voir le schéma de la figure 1).

Les propriétés du produit obtenu selon les exemples 1 et 2 sont les suivantes :

| | VRE | VRS | Capacité d'absorption solution saline | indice de défibrabilité (secondes) | taux de greffage de l'acrylonitrile |
|---|---|---|---|---|---|
| Superabsorbant | 45 | 20 | 20 | 5 | 200 % |

La vitesse d'absorption de la solution saline est de 18 g par minute et par gramme de produit.

**Revendications**

1. Procédé de préparation d'un produit fibreux et sec à base de cellulose modifiée ayant une rétention d'eau et une rétention de liquide physiologique améliorées par greffage sur une pâte cellulosique d'un monomère polymérisable à insaturation oléfinique, ayant des groupements fonctionnels hydrolysables, selon lequel on active la cellulose contenue dans la pâte cellulosique, on greffe le monomère sur la cellulose, on hydrolyse la pâte cellulosique greffée avec un alcali, on lave le produit à l'eau jusqu'à obtention d'un état de gonflement maximum, on acidifie le produit à un pH lui donnant après élimination d'eau, son état de gonflement minimum, on transforme le produit en sa forme sel en présence d'un liquide miscible à l'eau et on le sèche, caractérisé en ce que la siccité de la cellulose au début du greffage, avant introduction du monomère polymérisable est d'au moins 20 % environ, pour obtenir un taux de greffage de l'ordre de 200 %, la transformation du produit en sa forme sel étant conduite sous une agitation suffisante pour éviter l'agglomération des fibres entre elles, et on fait en sorte que, juste avant l'étape de séchage, la quantité d'eau n'excède pas environ 10 % en volume de la phase liquide.

2. Procédé selon la revendication 1, caractérisé en ce que pour que la quantité d'eau exprimée en volume, n'excède pas 10 % environ avant l'étape de séchage, on effectue un échange complet du liquide réactionnel avec un liquide miscible à l'eau.

3. Procédé selon la revendication 1, caractérisé en ce que pour que la quantité d'eau, exprimée en volume, n'excède pas 10 % environ de la phase liquide avant l'étape de séchage, on déplace l'eau acidifiée à l'aide d'un liquide miscible à l'eau, puis on dilue le milieu réactionnel avec ledit liquide miscible à l'eau.

4. Procédé selon l'une des revendications 1 à 3, caractérisé en ce que le monomère polymérisable est choisi parmi les monomères aptes au greffage sur de la cellulose et présentant une meilleure aptitude au greffage sur la cellulose qu'à la formation d'homopolymère dans le milieu considéré.

5. Procédé selon la revendication 4, caractérisé en ce que le monomère polymérisable est l'acrylonitrile.

6. Procédé selon l'une des revendications 1 à 5, caractérisé en ce que on active la cellulose avec un oxydant décomposable à la chaleur.

7. Procédé selon la revendication 6, caractérisé en ce que l'oxydant utilisé est le persulfate de sodium, d'ammonium ou de potassium.

8. Procédé selon l'une des revendications 6 ou 7, caractérisé en ce que on élimine l'oxydant décomposable à la chaleur après l'étape de greffage et avant l'étape d'hydrolyse.

9. Procédé selon l'une des revendications 1 à 8, caractérisé en ce que la transformation du produit en sa forme sel se fait avec de l'ammoniaque.

10. Procédé selon la revendication 9, caractérisé en ce que on récupère l'ammoniaque dégagé lors de l'hydrolyse de la pâte cellulosique greffée, on l'utilise pour transformer le produit en sa forme sel et on l'élimine au cours du séchage.

11. Procédé selon l'une des revendications 1 à 10, caractérisé en ce que le liquide miscible à l'eau est un alcool choisi parmi les alcools méthylique, éthylique, isopropylique.

12. Procédé de préparation d'un produit selon la revendication 1 selon lequel on active une pâte cellulosique avec du persulfate d'ammonium, on greffe de l'acrylonitrile sur la cellulose alors qu'elle présente une siccité d'au moins 20 % environ, on hydrolyse la pâte cellulosique greffée avec de la soude, on lave les fibres greffées et hydrolysées à l'eau jusqu'à obtention d'une siccité de 2,5 %, on acidifie jusqu'à un pH de 3, on essore le produit acidifié jusqu'à atteindre une siccité de 20 à 25 %, on déplace l'eau acidifiée à l'aide d'éthanol, on neutralise par introduction d'ammoniaque tout en agitant, on ajoute de l'éthanol pour former une feuille continue que l'on sèche.

13. Produit fibreux obtenu par la mise en œuvre du procédé selon une des revendications 1 à 12.

**Claims**

1. Process for the preparation of a fibrous, dry product based on modified cellulose having improved water retention and physiological liquid retention by grafting on a cellulose paste a polymerizable monomer with olefinic unsaturation, having hydrolyzable functional groups, according to which the cellulose contained in the cellulose paste is activated, the monomer is grafted to the cellulose, the grafted cellulose paste is hydrolyzed with an alkali, the product is washed with water until a maximum swelling state is obtained, the product is acidified to a pH-value which, after water elimination, gives it its minimum swelling state, the product is transformed into its salt form in the presence of a water-miscible liquid and is dried, characterized in that the dryness of the cellulose at the start of grafting and prior to the introduction of the polymerizable monomer is at least approximately 20 %, in order to obtain a grafting rate of approximately 200 %, the transformation of the product into its salt form being carried out under an adequate stirring to prevent agglomeration between one another of the fibres and the procedure is performed in such a way that, just prior to the drying stage, the water quantity does not exceed approximately 10 % by volume of the liquid phase.

2. Process according to claim 1, characterized in that in order that the water quantity expressed in

7

EP 0 188 146 B1

volume does not exceed approximately 10 % prior to the drying stage, there is a complete exchange of the reaction liquid with a water-miscible liquid.

3. Process according to claim 1, characterized in that for the water quantity expressed in volume not to exceed approximately 10 % of the liquid phase prior to the drying stage, the acidified water is displaced with the aid of a water-miscible liquid and then the reaction medium is diluted with said water-miscible liquid.

4. Process according to one of the claims 1 to 3, characterized in that the polymerizable monomer is chosen from among the monomers suitable for grafting to cellulose and having a better suitability for grafting to cellulose than for the formation of a homopolymer in the considered medium.

5. Process according to claim 4, characterized in that the polymerizable monomer is acrylonitrile.

6. Process according to one of the claims 1 to 5, characterized in that the cellulose is activated with a heat-decomposable oxidizing agent.

7. Process according to claim 6, characterized in that the oxidizing agent used is sodium, ammonium or potassium persulphate.

8. Process according to one of the claims 6 or 7, characterized in that the heat-decomposable oxidizing agent is eliminated after the grafting stage and prior to the hydrolysis stage.

9. Process according to one of the claims 1 to 8, characterized in that the transformation of the product into its salt form takes place with ammonia.

10. Process according to claim 9, characterized in that the ammonia given off during the hydrolysis of the grafted cellulose paste is recovered and is used for transforming the product into its salt form and it is eliminated during drying.

11. Process according to one of the claims 1 to 10, characterized in that the water-miscible liquid is an alcohol chosen from among methyl, ethyl and isopropyl alcohols.

12. Process for the preparation of a product according to claim 1, in which a cellulose paste is activated with ammonium persulphate, acrylonitrile is grafted on to the cellulose whilst it has a dryness of at least approximately 20 %, the grafted cellulose paste is hydrolyzed with soda, the grafted and water-hydrolyzed fibres are washed until a dryness of 2.5 % is obtained, followed by acidification to a pH of 3, suction filtering of the acidified product until a dryness of 20 to 25 % is obtained, the displacement of the acidified water with the aid of ethanol, neutralization by ammonia introduction accompanied by stirring and the addition of ethanol to form a continuous sheet, which is dried.

13. Fibrous product obtained by performing the process according to one of the claims 1 to 12.

**Patentansprüche**

1. Verfahren zum Herstellen eines faserförmigen und trockenen Produktes auf der Grundlage von Zellulose, die in Richtung einer besseren Speicherung von Wasser und physiologischer Flüssigkeit modifiziert worden ist durch Aufpfropfen eines olefinisch ungesättigten polymerisierbaren Monomers mit hydrolysierbaren funktionellen Gruppen auf eine Zellulosepaste, wobei man die in der Zellulosepaste enthaltene Zellulose aktiviert, das Monomer auf die Zellulose aufpfropft, die gepfropfte Zellulosepaste mit einem Alkali hydrolysiert, das Produkt mit Wasser bis zu einem maximal gequollenen Zustand wäscht, das Produkt bis zu einem pH ansäuert, der nach der Entfernung des Wassers einen Zustand minimaler Quellung ergibt, das Produkt in Gegenwart einer mit Wasser mischbaren Flüssigkeit in seine Salzform überführt und es trocknet, dadurch gekennzeichnet, daß der Trocknungsgrad der Zellulose zu Beginn des Aufpfropfens vor der Einführung des polymerisierbaren Monomers ungefähr mindestens 20 % beträgt, um einen Pfropfungsgrad von etwa 200 % zu erhalten und man die Umwandlung des Produktes in seine Salzform unter ausreichendem Rühren ausführt, um das Agglomerieren der Fasern aneinander zu verhindern und man diese Umwandlung so ausführt, daß unmittelbar vor der Trocknungsstufe die Wassermenge der flüssigen Phase etwa 10 Vol.-% nicht übersteigt.

2. Verfahren nach Anspruch 1, dadurch gekennzeichnet, daß man einen vollständigen Austausch der Reaktionsflüssigkeit mit einer mit Wasser mischbaren Flüssigkeit vornimmt, damit die Menge des Wassers vor der Trocknungsstufe etwa 10 Vol.-% nicht übersteigt.

3. Verfahren nach Anspruch 1, dadurch gekennzeichnet, daß man das angesäuerte Wasser durch eine mit Wasser mischbare Flüssigkeit ersetzt und dann das Reaktionsmedium mit der genannten mit Wasser mischbaren Flüssigkeit verdünnt, damit die Wassermenge vor der Trocknungsstufe etwa 10 Vol.-% der flüssigen Phase nicht übersteigt.

4. Verfahren nach einem der Ansprüche 1 bis 3, dadurch gekennzeichnet, daß das polymerisierbare Monomer aus zum Aufpfropfen auf Zellulose geeigneten Monomeren ausgewählt wird, und dieses Monomer eine größere Neigung zum Aufpfropfen auf der Zellulose aufweist als zur Bildung des Homopolymers im betrachteten Medium.

5. Verfahren nach Anspruch 1, dadurch gekennzeichnet, daß das polymerisierbare Monomer Acrylnitril ist.

6. Verfahren nach einem der Ansprüche 1 bis 5, dadurch gekennzeichnet, daß man die Zellulose mit einem in der Wärme zersetzbaren Oxidationsmittel aktiviert.

8

7. Verfahren nach Anspruch 6, dadurch gekennzeichnet, daß das benutzte Oxidationsmittel Natrium-, Ammonium- oder Kalium-persulfat ist.

8. Verfahren nach einem der Ansprüche 6 oder 7, dadurch gekennzeichnet, daß man das in der Wärme zersetzbare Oxidationsmittel nach dem Aufpfropfen und vor der Hydrolysestufe beseitigt.

9. Verfahren nach einem der Ansprüche 1 bis 8, dadurch gekennzeichnet, daß die Umwandlung des Produktes in seine Salzform mit Ammoniak erfolgt.

10. Verfahren nach Anspruch 9, dadurch gekennzeichnet, daß man das bei der Hydrolyse der gepfropften Zellulosepaste freigesetzte Ammoniak wiedergewinnt, es für die Umwandlung des Produktes in seine Salzform benutzt und es während des Trocknens beseitigt.

11. Verfahren nach einem der Ansprüche 1 bis 10, dadurch gekennzeichnet, daß die mit Wasser mischbare Flüssigkeit ein Alkohol ist, der ausgewählt ist aus Methyl-, Ethyl- und Isopropylalkohol.

12. Verfahren zum Herstellen eines Produktes nach Anspruch 1, bei dem man eine Zellulosepaste mit Ammoniumpersulfat aktiviert, Acrylnitril auf die Zellulose aufpfropft, während sie einen Trocknungsgrad von mindestens etwa 20 % hat, die gepfropfte Zellulosepaste mit Soda hydrolysiert, die gepfropften Fasern wäscht und mit Wasser hydrolysiert, bis man einen Trocknungsgrad von 2,5 % erhält, bis zu einem pH von 3 ansäuert, das angesäuerte Produkt bis zu einem Trocknungsgrad von 20 bis 25 % schleudert, das angesäuerte Wasser mittels Ethanol ersetzt, durch Zugabe von Ammoniak unter Rühren neutralisiert und Ethanol hinzufügt, um eine kontinuierliche Folie zu bilden, die man trocknet.

13. Faserförmiges Produkt erhalten gemäß dem Verfahren nach einem der Ansprüche 1 bis 12.

FIG_1

FIG_2

1